# NEW EUROPEAN PATENT SPECIFICATION

(11) **EP 1 371 379 B2**
(45) Date of publication and mention of the opposition decision: **20.06.2012**
(45) Mention of the grant of the patent: 25.03.2009
(21) Application number: 02012983.9
(22) Date of filing: 12.06.2002
(51) Int. Cl.: A61L 15/34, A61L 15/50, A61Q 19/00, A61Q 19/10

(54) **Absorbent article containing a skincare composition**
Absorbierender Artikel mit einer Hautpflegezusammensetzung
Article absorbant contenant une composition de soin pour la peau

(43) Date of publication of application: 17.12.2003
(73) Proprietor: SCA Hygiene Products AB, 405 03 Göteborg (SE)
(72) Inventor: Farbot, Anne, 436 39 Askim (SE); Runeman, Bo, 433 75 Jonsered (SE); Hjorth, Madeleine, 413 20 Göteborg (SE); Wild, Christine, D-40724 Hilden (DE); Mathis, Raymond, D-40627 Düsseldorf (DE); Neuss, Michael, D-50997 Köln (DE)
(74) Representative: Egeröd, Lisbeth

(56) References cited:
- EP-A2- 1 192 955
- WO-A-01/00129
- WO-A-96/16682
- WO-A1-01/22933
- DE-C- 3 309 530

## Description

### TECHNICAL FIELD

The present invention refers to an absorbent article such as a diaper, pant diaper, incontinence pad, sanitary napkin, pantiliner and the like. More particularly the present invention refers to absorbent articles having a skincare composition applied on at least a portion thereof, said composition being transferable to the skin of the wearer by normal contact and wearer motion and/or body heat.

### BACKGROUND OF THE INVENTION

In the preparation of hygiene articles, such as baby diapers or sanitary towels, absorbing materials are used in order to absorb aqueous fluids. In order to prevent direct contact with the absorbing material upon wearing and to increase the wear comfort, this material is covered with a thin, water-pervious nonwoven. Nonwoven materials of this type are usually prepared from synthetic fibres, such as polyolefin or polyester fibres, since these fibres can be produced at low cost, have good mechanical properties and are thermally stable.

In the hygiene article sector, nonwoven materials of this type are increasingly provided with skin-friendly lotions in order to generally improve tolerability and wear comfort. For example, DE 33 09 530 C1 describes a hygienic absorption liner, which is impregnated with a skincare material which consists of triglycerides and/or partial glycerides of coconut oil fatty acids having 8 to 18 carbon atoms. In order that these preparations can also transfer from the nonwoven to the skin without problems while being worn, the triglyceride and partial glyceride mixtures of DE 33 09 530 are chosen such that they have a rise point in the range from 35 to 40°C.

Another approach to transferring skincare substances to the skin during the wearing of hygiene articles is given in WO 96/16682. This describes a nappy whose inner covering web is prepared with a lotion which is solid or semisolid at 20°C and which transfers to the skin of the wearer while being worn. These lotions comprise from 10 to 95% of an anhydrous emollient, which has to be plastic or liquid at room temperature, and 5 to 90% of a so-called immobilizer which is to have a melting point of at least 35°C, but preferably 40°C.

However, the main problem of the known lotions is their storage stability. It is essential that the lotions themselves are in a form at skin temperature, i.e. approximately 36 to 38°C, such that they can transfer to the skin from the nonwoven without difficulties, i.e. at these temperatures the lotion should be sufficiently viscous to be detached from the nonwoven and transferred onto the skin. This temperature-dependent process can, however, lead to problems if the hygiene products are stored at relatively high temperatures, for example more than 30°C. In this case, it is frequently observed that the lotions "exude" on the nonwoven materials. It was therefore the object of the present invention to provide skin-friendly lotions for application to nonwoven materials for hygiene articles, where the storage stability of said lotions has to be ensured, in particular at high temperatures.

Furthermore, it is to be noted that the nonwoven in, for example, baby diapers must be pervious to liquids and has therefore usually been prepared to be hydrophilic. The further finishing with a usually hydrophobic skin-friendly lotion could therefore reduce or significantly impair the transportation of liquid through the web into the absorbing materials.

Furthermore, it is desired that the lotions transfer as completely as possible from the nonwoven onto the skin of the wearer and, in this connection, optionally provide further additional uses, for example are able to reduce the formation of an odour, or else the growth of bacteria, fungi and yeasts. In principle, it must of course be possible to apply the lotions to the nonwoven materials easily and to apply them as far as possible using the known preparation processes. It has been found that these properties could not be achieved in totality using the products of the prior art.

The lotion composition is typically applied to the absorbent article or a material intended to be used in such an article, in a molten state. This is normally done at a temperature between 35 and 100°C. Once the molten lotion has been applied it is allowed to cool and solidify to form solidified coating on the surface of the article or material onto which it has been applied. Any suitable application methods such as slot coating, extrusion coating, gravure coating and spraying methods may be used.

One main problem when applying lotion compositions to absorbent articles or a material intended to be used in such articles is that the manufacturing process often is a very rapid process and the molten lotion will not solidify sufficiently before the article or material is folded or rolled up, which results in smearing of the lotion. The lotion may further penetrate through for example the topsheet of the absorbent article, onto which it has been applied, and migrate into the absorbent core making this hydrophobic and less absorbent.

### SUMMARY OF THE INVENTION

It is an object of the present invention to provide an absorbent article containing a lotion of the above mentioned kind and which overcome or at least reduce the problems of smearing during the manufacturing of the article and penetration through the material onto which the lotion has been applied.

Surprisingly, it has been found that by combining three components chosen on the basis of their melting behaviour, it is possible to achieve the above object.

The present invention thus provides, in a first embodiment, absorbent article such as a diaper, pant diaper, adult incontinence guard, sanitary napkin and the like comprising a liquid permeable topsheet, a liquid impermeable backsheet and an absorbent body enclosed therebetween, said article having a skin treating composition applied on at least a portion thereof so as to be transferable to the skin of the wearer, said composition being solid at 21°C and comprises at least:
a) 5 to 70% by weight of a component having a melting point in the range from 25 to 37°C, chosen from the group of synthetic waxes, paraffins, fatty acid esters, polyhydroxy fatty acid esters, fatty alcohols, alkoxylated fatty acid esters, alkoxylated fatty alcohols and mixtures of these compounds and
b) 5 to 70% by weight of a component whose melting point is at least 5°C higher than the melting point of component a), and component b) is chosen from the group of paraffins, polyhydroxy fatty acid esters, C₁₄-C₂₂-fatty alcohols, C₁₂-C₂₂-fatty acids, the alkoxylated derivatives of the fatty alcohols and fatty esters, and mixtures of these components, where the compositions additionally comprise as component
c) 5 to 25% by weight of a crystallization accelerator in the form of a polyvinyl stearyl ether with the proviso that the crystallization accelerator has a melting point in the range from 45 to 70°C, and that the compositions comprise less than 5% by weight of water.

The compositions according to the invention obligatorily comprise three constituents, being characterized, in particular, by the presence of component c), a crystallization accelerator. Furthermore, it is essential to chose components a) to c) according to their melting behaviour.

It is preferred that component b) has a melting point in the range from 40 to 60°C.

In one aspect of the invention component a) is chosen from the mixtures of glycerides of fatty acids having 8 to 18 carbon atoms, preferably from technical-grade mixtures of partial glycerides and/or mixtures with glycerides.

In a further aspect of the invention component b) is chosen from mixtures of glycerides of fatty acids having 8 to 18 carbon atoms, preferably from technical-grade mixtures of partial glycerides and/or mixtures with glycerides.

Component a) and/or component b) may in one embodiment each comprise glycerol triesters or partial esters of coconut fatty acids, the mixtures in each case having a melting point in the claimed range.

Preferably the skincare composition comprises component a) in amounts of from 10 to 60% by weight, component b) in amounts of from 10 to 60% by weight and component c) in amounts of from 10 to 25% by weight.

It is further preferred that the skincare composition has a melting point in the range from 35 to 65°C, preferably from 35 to 50°C and in particular from 35 to 45°C.

The skincare composition may further comprise silicone waxes in amounts of from 1 to 6% by weight, preferably 1.5 to 5.5% by weight and in particular from 2 to 5% by weight.

The skincare composition may further comprise skin-friendly and/or skincare substances in amounts of from 0.1 to 10% by weight, preferably 1 to 8% by weight and in particular from 2 to 6% by weight.

In one aspect of the invention the skincare composition comprises water in amounts of from 0.5 to 3% by weight, preferably 0.5 to 2% by weight and in particular 0.5 to 1.5% by weight.

In one embodiment the skincare composition comprises 50 to 60% by weight of a mixture of glycerol esters of coconut fatty acids having a melting point of from 30 to 33°C as component a), 10 to 20% by weight of a linear, unsaturated fatty alcohol having a melting point of from 57 to 60°C as component b), 15 to 20 parts of polyvinyl stearyl ether having a melting point of from 45 to 48°C as component c), and optionally 2 to 5% by weight of silicone wax, and 5 to 10% by weight of a skincare substance.

In one embodiment the skincare composition is applied on at least part of the topsheet of the absorbent article. Different amounts of skincare composition may be applied in different zones of the topsheet. For example the intended wetting region of the topsheet material may contain no or a smaller amount of skincare composition as compared to the surrounding regions of the topsheet material.

In still a further embodiment the skincare composition is applied on any material and component of the article, such as elastic members, belts, fibers etc., which during use of the article is in contact with the skin of the wearer via for example the liquid pervious topsheet.

In a further aspect of the invention at least two different skincare compositions are applied in different regions of the article.

### DESCRIPTION OF THE DRAWINGS

Fig. 1 is a perspective view of an absorbent article in the form of a diaper.
Fig. 2 is a fragmentary sectional view through an absorbent article.

### DETAILED ESCRIPTION OF THE INVENTION

The term "absorbent article" refers to products that are placed against the skin of the wearer to absorb and contain body exudates, like urine, faeces and menstrual fluid. The invention mainly refers to disposable absorbent articles, which means articles that are not intended to be laundered or otherwise restored or reused as an absorbent article after use. Examples of disposable absorbent articles include feminine hygiene products such as sanitary napkins, pantiliners and sanitary panties; diapers and pant diapers for infants and incontinent adults; incontinence pads; diaper inserts and the like.

The drawing shows an embodiment of a diaper 1 for an infant or an incontinent adult, said diaper typically comprises a chassis comprising a liquid permeable topsheet 2, a liquid impermeable backsheet 3 and an absorbent body 4 enclosed therebetween. The liquid permeable topsheet 2 can consist of a nonwoven material, e g spunbonded, meltblown, carded, hydroentangled, wetlaid etc. Suitable nonwoven materials cab be composed of natural fibers, such as woodpulp or cotton fibres, manmade fibres, such as polyester, polyethylene, polypropylene, viscose etc. or from a mixture of natural and manmade fibres. The topsheet material may further be composed of tow fibres, which may be bonded to each other in a bonding pattern, as e.g. disclosed in EP-A-1 035 818. Further examples of topsheet materials are porous foams, apertured plastic films etc. The materials suited as topsheet materials should be soft and non-irritating to the skin and be readily penetrated by body fluid, e g urine or menstrual fluid.

The liquid impermeable backsheet 3 may consist of a thin plastic film, e.g. a polyethylene or polypropylene film, a nonwoven material coated with a liquid impervious material, a hydrophobic nonwoven material, which resists liquid penetration or laminates of plastic films and nonwoven materials. The backsheet material may be breathable so as to allow vapour to escape from the absorbent core, while still preventing liquids from passing through the backsheet material.

The topsheet 2 and the backsheet material 3 have a somewhat greater extension in the plane than the absorbent body 4 and extend outside the edges thereof. The layers 2 and 3 are connected to each other within the projecting portions thereof, e g by gluing or welding by heat or ultrasonic. The topsheet and/or the backsheet may further be attached to the absorbent core by any method known in the art, such as adhesive, heatbonding etc. The absorbent core may also be unattached to the topsheet and/or the backsheet.

The absorbent body 4 can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsor-bents in an absorbent body. It is also common to have absorbent bodies comprising layers of different material with different properties with respect to liquid acquisition capacity, liquid distribution capacity and storage capacity. This is well-known to the person skilled in the art and does therefore not have to be described in detail. The thin absorbent bodies, which are common in for example baby diapers and incontinence guards, often comprise a compressed mixed or layered structure of cellulosic fluff pulp and superabsorbent. The size and absorbent capacity of the absorbent core may be varied to be suited for different uses such as baby diapers, adult incontinence diapers and pads, pant diapers, pantiliners, sanitary napkins etc.

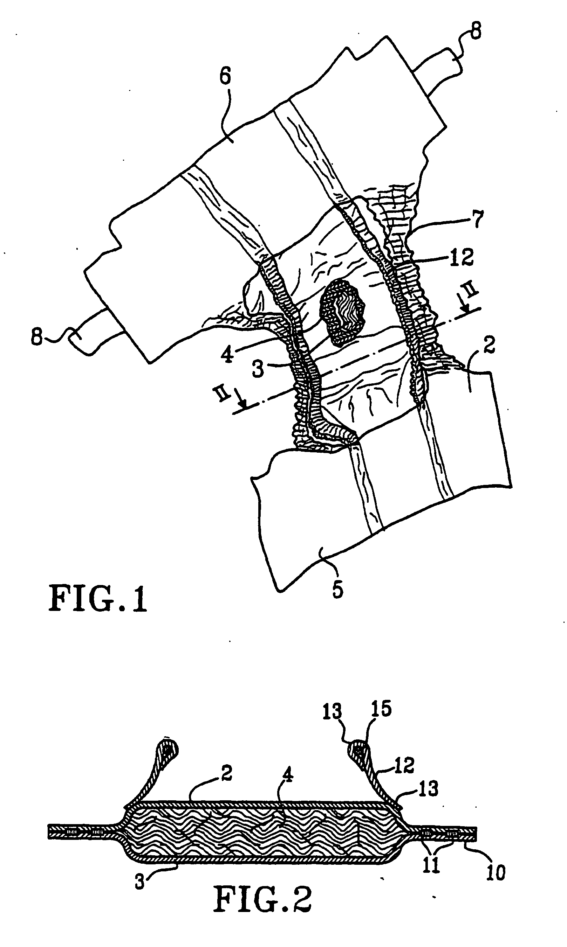

The diaper disclosed in Fig. 1 is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs. The front portion 5 is provided with a pair of adhesive tape tabs 8 or other type of attachment means such as hook-and-loop type fasteners.

The diaper comprises elasticised side flaps 10 forming leg openings. Elastification is provided by elastic members 11 secured between the topsheet and backsheet in the side flap region 10. The diaper disclosed in Fig. 1 and 2 further comprises elastic barrier flaps 12 having a proximal edge 13 and a distal edge 14 and elastic member 15 spacing the distal edge 14 away from the topsheet. These barrier flaps 12 form leakage barriers and are at their proximal edges 13 secured to the topsheet 2 close to the lateral edges of the absorbent core 4 either in the area of the side flaps 10 or above the absorbent core 4.

The diaper may further comprise elasticised waist feature in the form of elastic members extending in the transverse direction of the article in the wais portion thereof.

In a further embodiment the diaper comprises belt portions attached to the rear portion of the diaper and intended to be fastened together around the waist of the wearer. Fastening means on the front part of the diaper are then attached to the outside of the belt to fasten together the diaper to the desired pantlike shape. An example of a belted diaper is shown in WO 01/00129.

It is however understood that the diaper described above and shown in the drawing only represents one non-limiting example and that the present invention is not limited thereto, but can be used in any type of absorbent articles as defined above.

The absorbent article according to the present invention has a skin care composition applied on at least a portion thereof so as to be transferable to the skin of the wearer by normal contact, wearer motion and/or body temperature. When transferred to the skin the skin care composition provide desirable skin treating or protective benefits such as reduced red marking, erythema, diaper dermatitis and skin irritation.

Several factors in combination lead to the development of diaper dermatitis or diaper rash. Wet skin results in that chafing and pressure more easily wear down the skin. A high moisture content also means that skin penetration by irritant substances can increase, and that bacteria and fungi can thrive. Occlusion of skin and breakdown of urea in the urine to ammonia results in an increase in the pH. The higher pH value leads to that enzymes (lipases and proteases) coming from the intestine, and from the microorganisms in the excrement, can break down the skin to a greater extent. A vicious circle can easily develop in which various factors facilitate and intensify each other.

Dermatitis is best prevented by creating conditions, which counteract those factors which create and maintain the process of diaper dermatitis. It should therefore be endeavoured to keep the skin as dry as possible, to air the skin often and to change wet diapers. Mechanical shearing forces should be minimized by choosing materials, which are as smooth and soft as possible, and wear between diaper and skin should be minimized. By supplying the skin with a softening and protective lotion or cream, it is further possible to strengthen the barrier against penetration of irritant substances and enzymes. In more serious cases of dermatitis, microorganisms may have infected the damaged skin, and treatment with more active medicines is required. Ointment with cortisone and various fungicidal and bactericidal agents are then used.

The skin care composition may be applied to any portion of the article that during use is in contact with the skin of the wearer either directly or through the pervious topsheet. Such portions include the topsheet 2 or part thereof, the elasticised side flaps 10, the barrier flaps, the belts in a belted diaper, the elastic members in the side flaps, barrier flaps and/or waist portion. The skin care composition may also be applied to fibers contained in a nonwoven material and tow fibres in a tow material.

The skin care composition according to the invention comprises being solid at 21°C and comprises at least:
a) 5 to 70% by weight of a component having a melting point in the range from 25 to 37°C, chosen from the group of synthetic waxes, paraffins, fatty acid esters, polyhydroxy fatty acid esters, fatty alcohols, alkoxylated fatty acid esters, alkoxylated fatty alcohols and mixtures of these compounds and
b) 5 to 70% by weight of a component whose melting point is at least 5°C higher than the melting point of component a), and component b) is chosen from the group of paraffins, polyhydroxy fatty acid esters, C₁₄-C₂₂-fatty alcohols, C₁₂-C₂₂-fatty acids, the alkoxylated derivatives of the fatty alcohols and fatty esters, and mixtures of these components, where the compositions additionally comprise as component
c) 5 to 25% by weight of a crystallization accelerator in the form of a polyvinyl stearyl ether with the proviso that the crystallization accelerator has a melting point in the range from 45 to 70°C, and that the compositions comprise less than 5% by weight of water.

Melting itself is defined as the transition of a substance from the solid to the liquid aggregate state by the input of thermal energy, where, as a consequence of an increase in kinetic energy of the particles, their oscillation amplitude becomes so great that the lattice structure collapses. The melting point is defined as the temperature at which the liquid and the solid phase of a substance are in thermodynamic equilibrium at a pressure of, normally, 1013 kPa. In actual fact, the term "melting point" is, however, used in practice mostly only for the transition point from the solid state to the liquid state, and not for the temperature, identical thereto, at which the transition in the reverse direction takes place. The amount of heat absorbed in this process is referred to as the heat of melting or the enthalpy of melting. Usually, the melting point increases with increasing pressure, although there are exceptions. For many pure substances, the melting points can be determined with great accuracy since here the temperature remains constant over a certain time interval during the introduction of heat. For amorphous, glass-like substances, there is no specific melting point since there are no crystal lattices here. Similar phenomena are observed in the melting behaviour of fats, ointments, creams or suppository materials; in such cases, it is possible to use the solidification point, the so-called rise point and the dropping point for characterizations.

The action of this crystallization accelerator is to be understood as meaning it has a very sharp melting point which must be higher than the melting point of the liquid to semiliquid component a), but should be lower than the temperature at which the lotion is applied. The combination of component a), which melts at relatively low temperatures, with component b), which melts at higher temperatures, and the simultaneous addition of the crystallization accelerator c) gives lotions which, firstly, are still storage-stable even at high temperatures, and at the same time can be transferred from the article to the skin of the wearer while being worn.

Component a) can be chosen from a large number of compounds known to the person skilled in the art, it being essential that the melting point here must be in the range from 25 to at most 37°C. Firstly, for this purpose it is possible to use certain paraffins, but also fatty acid esters and, in particular, fatty alcohols. Suitable paraffins are preferably semisolid paraffins, such as soft paraffin, preferably petrolatum. Suitable fatty alcohols are, for example, dodecanol or ricinol alcohol, to name one representative of the unsaturated fatty alcohols. Further suitable substances are chosen from the class of synthetic waxes, for example copolymers of polyethylene/maleic anhydride.
For the purposes of the present invention, the use of glycerides is particularly suitable, here preferably the mixtures of partial glyceride and triglycerides, which must have the desired melting point of from 25 to 37°C. Particular preference is given here to mixtures of glycerides of fatty acids having 8 to 18 carbon atoms.

Glycerides are mono-, di- and/or triesters of glycerol with fatty acids, namely, for example, caproic acid, caprylic acid, 2-ethylhexanoic acid, capric acid, lauric acid, isotridecanoic acid, myristic acid, palmitic acid, palmoleic acid, stearic acid, isostearic acid, oleic acid, elaidic acid, petroselic acid, linoleic acid, linolenic acid, elaeostearic acid, arachidic acid, gadoleic acid, behenic acid and erucic acid, and technical-grade mixtures thereof. They conform to the formula (I), 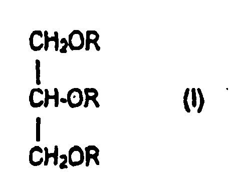 in which R is a COR' radical, in which R' is a branched or unbranched, saturated or unsaturated alkyl radical having 6 to 22 carbon atoms, and/or independently thereof, is hydrogen. Typical examples are lauric acid monoglyceride, lauric acid diglyceride, coconut fatty acid monoglyceride, coconut fatty acid triglyceride, palmitic acid monoglyceride, palmitic acid triglyceride, stearic acid monoglyceride, stearic acid diglyceride, isostearic acid monoglyceride, isostearic acid diglyceride, oleic acid monoglyceride, oleic acid diglyceride, tallow fatty acid monoglyceride, tallow fatty acid diglyceride, behenic acid monoglyceride, behenic acid diglyceride, erucic acid monoglyceride, erucic acid diglyceride, and technical-grade mixtures thereof, which may also comprise small amounts of triglyceride as secondary components from the preparation process.

Suitable as component a) are, in particular, those mixtures of coconut partial glyceride and triglycerides sold under the trade name Novata^{®}-B by the applicant. Novata^{®}-B has a melting point (in accordance with DGF C-IV 3a) of from 33 to 36°C, and the acid number is 0.3 (in accordance with DGF C-V2). The saponification value is 225-240 in accordance with DGF C-V 3. The molecular weight is 710. A further suitable mixture for component a) is Novata^{®}-299 (melting point 34°C) from the applicant.

Component b) must have a melting point which is at least 5°C higher than the melting point of component a) used in the composition in question. Particular preference is given here to those compounds of the group of C₁₄₋₂₂-fatty alcohols, C₁₂₋₂₂-fatty acids and alkoxylated derivatives thereof, and fatty alcohols and esters. However, hard paraffins having the desired melt properties are also suitable. In this case too, the mixtures of different glycerides or fatty acids of the C₈₋₁₈ cut, in particular, are suitable as component b) for the purposes of the present technical teaching. Especially suitable both for component a) and also b) are glycerol triesters and partial esters of coconut fatty acids. These are the C₈-C₁₈ cut, where, depending on the choice of chain lengths of the glycerides or of the degree of esterification, it is possible to create mixtures with varying melting points. Mixtures of lauric (C₁₄) and myristic (C₁₆) esters are essentially present.

The higher-melting component b) used is preferably Novata^{®}-D from the applicant. This too is a mixture of triglycerides and partial glycerides of coconut fatty acids, but with a different melting range. Novata^{®}-D has a melting point (in accordance with DGF C-IV 3a) of from 40 to 42°C, and the acid number is 0.3 (in accordance with DGF C-V2). The saponification value is 215-230 (in accordance with DGF C-V 3).

The use of the crystallization accelerator component c) is essential for the present invention. This is a substance with a melting range which is, firstly, at least 45°C, and, secondly, the melting range of this component should, however, preferably be as narrow as possible and should thus not extend over more than 4.5°C, preferably 2.5°C, and below.

A polyvinyl stearyl ether is used as the crystallization accelerator component c).

If the crystallization accelerator c) according to the invention is used, lotions are obtained which can be applied to the article or to a material intended to be used therein, such as a topsheet nonwoven material, elastic members or the like without problems since their melting temperature is higher than that of the lower-melting component a), but is not so high that it reaches the application temperature of the lotion onto the article or material. The application temperature is preferably 60-80°C, it being necessary to also adapt the application temperature, depending on the crystallization accelerator used.

In selecting components a) to c), their melting points are, firstly of significance. In addition, it is to be taken into consideration that at least three different substances are present in the composition according to the invention according to the definition of component a) to c). It is particularly preferred if component c) has a melting point below the melting point of component b) and is preferably 5 to 10°C, in particular 10 to 15°C, lower.

The compositions themselves preferably have a melting point in the range from 35 to 65°C, in particular from 35 to 50°C and preferably 35 to 45°C. The melting behaviour of the compositions is essential for the use, and it is therefore preferred for the compositions according to the invention to have two melting ranges, which are clearly separate from one another, as can preferably be ascertained by means of TLC measurements. The enthalpies of melting for the compositions are preferably 80 to 160 J/g, in particular 90 to 140 J/g and particularly preferably 100 to 125 J/g. Furthermore, the co-use of liquid or semisolid compounds, as disclosed in the above-cited WO 96/16682, and also in WO 96/16681, is excluded.

The compositions according to the invention are anhydrous, i.e. they comprise water in a total amount of 5% by weight, preferably 0.5 to 3% by weight and in particular 0.1 to 2.0% by weight. Accordingly, anhydrous components a) to c) are preferably to be chosen in order to avoid expensive drying steps during the preparation.

In the compositions according to the invention, in a preferred embodiment, component a) is present in amounts of from 10 to 60% by weight, component b) is present in amounts of from 10 to 60% by weight and component c) is present in amounts of from 10 to 30% by weight.

In addition, further customary ingredients may also be present in the compositions according to the invention, for example silicone waxes or polysiloxanes, in amounts of from 1 to 6% by weight, preferably 1.5 to 5.5% by weight and in particular from 2 to 5% by weight. Polysiloxanes are known polymeric compounds, which contain the following structure as monomer units:

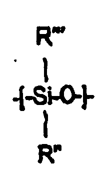

Here, R" and R"', independently of one another, are hydrogen or an alkyl, cycloalkyl, aryl or alkenyl radical. Siloxanes of this type preferably have viscosities at 37°C in the range from 5 to 5 000 mPa s.

In addition, the compositions according to the invention may comprise skin-friendly or skincare substances, preferably in amounts of from 0.1 to 10% by weight, in particular 1 to 8% by weight and very particularly preferably from 2 to 6% by weight.

Ingredients of this type may, for example, be bisabolol, alantoin and panthenol. It is also possible to use vitamins, preferably vitamin E and vitamin precursors, and protein hydrolysates. Also suitable are plant extracts, preferably from camomile, aloe vera, lime blossom, horse chestnut, green tea, oak bark, stinging nettle, hops, burdock, horsetail, hawthorn, almond, spruce needle, almond wood, juniper, coconut, apricot, lemon, wheat, kiwi, melon, orange, grapefruit, sage, rosemary, birch, mallow, lady's smock, thyme, balm, restharrow, coltsfoot, althea, ginseng and root ginger. In addition, however, other skincare substances may also be present. Those skincare compositions, which may be named here are, in particular, chitosan, and zinc oxide or zinc ricinoleate.

A particularly preferred composition of the present invention comprises 50 to 60% by weight of a mixture of glycerol esters of coconut fatty acids having a melting point of from 30 to 33°C as component a), 10 to 20% by weight of a linear, unsaturated fatty alcohol having a melting point of from 57 to 60°C as component b), 15 to 20 parts of polyvinyl stearyl ether having a melting point of from 45 to 48°C as component c), and optionally 2 to 5 % by weight of silicone wax, and optionally 5 to 10% by weight of a skincare substance, preferably of avocado oil.

The compositions are prepared in a manner customary per se, by mixing the individual liquid components, i.e. at elevated temperatures, preferably at 40 to 80°C and in particular at 50 to 70°C. A particular sequence during the mixing of the components is not necessary. The compositions are then cooled to room temperature (21°C).

Since the skincare composition according to the invention solidifies rapidly, it provides several advantages in the manufacturing process of absorbent articles, which takes place in a very high speed, several hundreds of articles per minute, or in the case of the production of nonwoven web materials, several hundreds of meters per minute. Due to the rapid solidification the composition, which is applied in a molten state, will solidify sufficiently before the article or material is folded or rolled up, so that smearing of the molten composition is avoided or at least reduced. The risk that the composition may penetrate through for example the topsheet of the absorbent article, onto which it has been applied, and migrate into the absorbent core making this hydrophobic and less absorbent is further reduced or even avoided. More composition may further be applied on the article or material in the process since the solidification process is more rapid.

The skincare composition is preferably applied to for example the topsheet material or any other material or component of an absorbent article, before said material or component is joined to the other components of the article. It may alternatively be applied to the absorbent article after the materials and components contained therein have been joined together. It may be applied by any useful technique known in the art, such as spraying, printing, slot-nozzle application etc. It may be continuously applied or in discrete areas, in gradients or patterns.

The skincare composition may be applied in different amounts in different parts of the topsheet material. For example the intended wetting region of the topsheet, which is the region which during use of the article is located in the crotch region where the discharged body fluid will be received, may contain no or a relatively smaller amount of skincare composition as compared to the regions of the topsheet material located outside the wetting region.

Two or more skincare compositions may further be applied in different regions of the article. Thus for example a skin composition especially adapted to alleviate red markings caused by pressure may be used in the regions of the elastic members in the side flaps, barrier flaps and/or waist portion, while another skin composition especially adapted to alleviate diaper rash caused by skin irritation from urine and/or faeces, may be applied on the topsheet material in those areas which will be wetted by body discharges. Thus by using the skincare compositions disclosed herein there is provided a great freedom in choosing where and how the composition may be applied to the article or a material intended to be incorporated in an absorbent article.

### Examples

The following compositions 1 and 2 according to the invention were prepared and their melting behaviour was investigated. For this purpose, DSC measurements were carried out in each case. The heating/Cooling rates were 10K/min and -1 K/min respectively.

### Composition 1:

a) 58% by weight of a mixture of partial glycerides and triglycerides of coconut fatty acids, melting point: 34°C
b) 15% by weight of stearyl alcohol, melting point 56-58°C
c) 20% by weight of polyvinyl stearyl ether, melting point 45°C
d) 2% by weight of silicone wax

The melting point of the composition was 49°C. The heat of melting was 112 J/g.

### Composition 2:

a) 55% by weight of a mixture of partial glycerides and triglycerides of coconut fatty acids, melting point: 34°C
b) 15% by weight of glycerol monolaurate, melting point 63°C
c) 20% by weight of polyvinyl stearyl ether, melting point 45°C
d) 5% by weight of silicone wax

The melting point was 46°C. The heat of melting was 82 J/g.

The compositions described above can be applied to nonwovens without problems and are suitable for the preparation of nonwovens for hygiene products.

To determine the cooling characteristic of lotions according to the present invention three lotions were prepared and heated to 70 °C. Lotions 1 and 2 (corresponding to the above compositions 1 and 2) contain crystallisation inhibitors according to the invention. Lotion 3 (corresponding to composition 1 above) is free of this compound.

The hot lotions were put (0.5 ml each) on a slanting glass plate (angle 35°). Then the distance was measured, until the drop comes to stop. For lotion 1 and 2 a distance of approximately 15 cm was measured the lotion without compound c) according to claim 1 needs 21 cm to come to stop.

## Claims

1. An absorbent article such as a diaper, pant diaper, adult incontinence guard, sanitary napkin and the like comprising a liquid permeable topsheet (2) a liquid impermeable backsheet (3) and an absorbent body (4) enclosed therebetween, said article having a skincare composition applied on at least a portion thereof so as to be transferable to the skin of the wearer, said composition being solid at 21°C and comprises at least:
a) 5 to 70°rb by weight of a component melting in the range from 25 to 37°C, chosen from the group of synthetic waxes, paraffins, fatty acid esters, polyhydroxy fatty acid esters, fatty alcohols, alkoxylated fatty acid esters, alkoxylated fatty alcohols and mixtures of these compounds and
b) 5 to 70% by weight of a component whose melting point is at least 5°C higher than the melting point of component a), and component b) is chosen from the group of paraffins, polyhydroxy fatty acid esters, C₁₄-C₂₂-fatty alcohols, C₁₂-C₂₂-fatty acids, the alkoxylated derivatives of the fatty alcohols and fatty esters, and mixtures of these components,
**characterized in**
**that** the composition additionally comprises as component
c) 5 to 25% by weight of a crystallization accelerator in the form of a polyvinyl stearyl ether, with the proviso that the crystallization accelerator has a melting point in the range from 45 to 70°C,
and **that** the compositions comprise less than 5% by weight of water.

2. Absorbent article according to claim 1, **characterized in that** component b) has a melting point in the range from 10 to 60°C.

3. Absorbent article according to claim 1 or 2, **characterized in that** component a) is chosen from the mixtures of glycerides of fatty acids having 8 to 18 carbon atoms, preferably from technical-grade mixtures of partial glycerides and/or mixtures with glycerides.

4. Absorbent article according to any of the preceding claims, **characterized in that** component b) is chosen from mixtures of glycerides of fatty acids having 8 to 18 carbon atoms, preferably from technical-grade mixtures of partial glycerides and/or mixtures with glycerides.

5. Absorbent articles according to any of the preceding claims, **characterized in that** component a) and/or component b) each comprise glycerol triesters or partial esters of coconut fatty acids, the mixtures in each case having a melting point in the claimed range.

6. Absorbent article according to any of the preceding claims, **characterized in that** the skincare composition comprises component a) in amounts of from 10 to 60% by weight, component b) In amounts of from 10 to 60% by weight and component c) In amounts of from 10 to 25% by weight.

7. Absorbent article according to any of the preceding claims, **characterized in that** the skincare composition has a melting point in the range from 35 to 65°C, preferably from 35 to 50°C and In particular from 35 to 45°C.

8. Absorbent article according to any of the preceding claims, **characterized in that** the skincare composition comprises silicone waxes in amounts of from 1 to 6% by weight, preferably 1.5 to 5.5% by weight and In particular from 2 to 5% by weight.

9. Absorbent article according to any of the preceding claims, **characterized in that** the skincare composition comprises skin-friendly and/or skincare substances in amounts of from 0.1 to 10% by weight, preferably 1 to 8% by weight and In particular from 2 to 6% by weight.

10. Absorbent article according to any of the preceding claims, **characterized in that** the skincare composition comprises water in amounts of from 0.5 to 3% by weight, preferably 0.5 to 2% by weight and In particular 0.5 to 1.5% by weight.

11. Absorbent article according to any of the preceding claims, **characterized in that** the skincare composition comprises 50 to 60% by weight of a mixture of glycerol esters of coconut fatty acids having a melting point of from 30 to 33°C as component a), 10 to 20% by weight of a linear, unsaturated fatty alcohol having a melting point of from 57 to 60°C as component b), 15 to 20 parts of polyvinyl stearyl ether having a melting point of from 45 to 48°C as component c), and optionally 2 to 5% by weight of silicone wax, and 5 to 10% by weight of a skincare substance.

12. Absorbent article according to any of the preceding claims, **characterized in that** the skincare composition is applied on at least part of the topsheet (2) of the article.

13. Absorbent article according to claim 12, **characterized in that** different amounts of skincare composition are applied in different zones of the topsheet.

14. Absorbent article according to claim 13, **characterized in that** the intended wetting region of the topsheet material contains no or a smaller amount of skincare composition as compared to the surrounding regions of the topsheet material.

15. Absorbent article according to any of the preceding claims, **characterized in that** the skincare composition is applied on any material and component of the article, such as elastic members, belts, fibers etc., which during use of the article is in contact with the skin of the wearer via for example the liquid pervious topsheet.

16. Absorbent article according to any of the preceding claims, **characterized in that** at least two different skincare compositions are applied in different regions of the article.

## Patentansprüche

1. Absorbierender Artikel, wie eine Windel, eine Hosenwindel, ein Inkontinenzschutz für Erwachsene, eine Damenbinde und dergleichen, der ein flüssigkeitsdurchlässiges Topsheet (2), ein flüssigkeitsundurchlässiges Backsheet (3) und einen zwischen diese eingeschlossenen absorbierenden Körper (4) umfaßt, wobei der Artikel eine auf mindestens einen Teil davon applizierte Hautpflegezusammensetzung hat, so daß diese auf die Haut des Trägers transferierbar ist, wobei die Zusammensetzung bei 21°C fest ist und zumindest umfaßt:
a) 5 bis 70 Gew.% einer Komponente, die im Bereich von 25 bis 37°C schmilzt, ausgewählt aus der Gruppe aus synthetischen Wachsen, Paraffinen, Fettsäureestern, Polyhydroxyfettsäureestern, Fettalkoholen, alkoxylierten Fettsäureestern, alkoxylierten Fettalkoholen und Mischungen dieser Komponenten, und
b) 5 bis 70 Gew.% einer Komponente, deren Schmelzpunkt mindestens 5°C höher ist als der Schmelzpunkt der Komponente a), und die Komponente b) ausgewählt ist aus der Gruppe von Paraffinen, Polyhydroxyfettsäureestern, C₁₄-C₂₂-Fettalkoholen, C₁₂-C₂₂-Fettsäuren, den alkoxylierten Derivaten der Fettalkohole und Fettester und Mischungen dieser Komponenten,
**dadurch gekennzeichnet, daß** die Zusammensetzung zusätzlich als Komponente
c) 5 bis 25 Gew.% eines Kristallisationsbeschleunigers in Form eines Polyvinylstearylethers umfaßt,
mit der Maßgabe, daß der Kristallisationsbeschleuniger einen Schmelzpunkt im Bereich von 45 bis 70°C hat, und daß die Zusammensetzungen weniger als 5 Gew.% Wasser umfassen.

2. Absorbierender Artikel gemäß Anspruch 1, **dadurch gekennzeichnet, daß** die Komponente b) einen Schmelzpunkt im Bereich von 40 bis 60°C hat.

3. Absorbierender Artikel gemäß Anspruch 1 oder 2, **dadurch gekennzeichnet, daß** die Komponente a) ausgewählt ist aus den Mischungen von Glyceriden von Fettsäuren mit 8 bis 18 Kohlenstoffatomen, vorzugsweise aus Mischungen technischen Grades von Partialglyceriden und/oder Mischungen mit Glyceriden.

4. Absorbierender Artikel gemäß einem der vorhergehenden Ansprüche, **dadurch gekennzeichnet, daß** die Komponente b) ausgewählt ist aus Mischungen von Glyceriden von Fettsäuren mit 8 bis 18 Kohlenstoffatomen, vorzugsweise aus Mischungen technischen Grades von Partialglyceriden und/oder Mischungen mit Glyceriden.

5. Absorbierender Artikel gemäß einem der vorhergehenden Ansprüche, **dadurch gekennzeichnet, daß** Komponente a) und/oder Komponente b) jeweils Glyceroltriester oder Partialester von Kokosnußfettsäuren umfassen, wobei die Mischungen in jedem Fall einen Schmelzpunkt in dem beanspruchten Bereich haben.

6. Absorbierender Artikel gemäß einem der vorhergehenden Ansprüche, **dadurch gekennzeichnet, daß** die Hautpflegezusammensetzung die Komponente a) in Mengen von 10 bis 60 Gew.%, die Komponente b) in Mengen von 10 bis 60 Gew.% und die Komponente c) in Mengen von 10 bis 25 Gew.% enthält.

7. Absorbierender Artikel gemäß einem der vorhergehenden Ansprüche, **dadurch gekennzeichnet, daß** die Hautpflegezusammensetzung einen Schmelzpunkt im Bereich von 35 bis 65°C, vorzugsweise von 35 bis 50°C und insbesondere von 35 bis 45°C hat.

8. Absorbierender Artikel gemäß einem der vorhergehenden Ansprüche, **dadurch gekennzeichnet, daß** die Hautpflegezusammensetzung Siliconwachse in Mengen von 1 bis 6 Gew.%, vorzugsweise 1,5 bis 5,5 Gew.% und insbesondere von 2 bis 5 Gew.% enthält.

9. Absorbierender Artikel gemäß einem der vorhergehenden Ansprüche, **dadurch gekennzeichnet, daß** die Hautpflegezusammensetzung hautfreundliche und/oder Hautpflegesubstanzen in Mengen von 0,1 bis 10 Gew.%, vorzugsweise 1 bis 8 Gew.% und insbesondere von 2 bis 6 Gew.% enthält.

10. Absorbierender Artikel gemäß einem der vorhergehenden Ansprüche, **dadurch gekennzeichnet, daß** die Hautpflegezusammensetzung Wasser in Mengen von 0,5 bis 3 Gew.%, vorzugsweise 0,5 bis 2 Gew.% und insbesondere von 0,5 bis 1,5 Gew.% enthält.

11. Absorbierender Artikel gemäß einem der vorhergehenden Ansprüche, **dadurch gekennzeichnet, daß** die Hautpflegezusammensetzung 50 bis 60 Gew.% einer Mischung von Glycerolestern von Kokosnußfettsäuren mit einem Schmelzpunkt von 30 bis 33°C als Komponente a), 10 bis 20 Gew.% eines linearen ungesättigten Fettalkohols mit einem Schmelzpunkt von 57 bis 60°C als Komponente b), 15 bis 20 Teile Polyvinylstearylether mit einem Schmelzpunkt von 45 bis 48°C als Komponente c) und optional 2 bis 5 Gew.% Siliconwachs und 5 bis 10 Gew.% einer Hautpflegesubstanz enthält.

12. Absorbierender Artikel gemäß einem der vorhergehenden Ansprüche, **dadurch gekennzeichnet, daß** die Hautpflegezusammensetzung auf mindestens einen Teil des Topsheets (2) des Artikels appliziert ist.

13. Absorbierender Artikel gemäß Anspruch 12, **dadurch gekennzeichnet, daß** unterschiedliche Mengen der Hautpflegezusammensetzung in verschiedenen Zonen des Topsheets appliziert sind.

14. Absorbierender Artikel gemäß Anspruch 13, **dadurch gekennzeichnet, daß** der Bereich des Topsheetmaterials, der befeuchtet werden soll, keine oder eine geringere Menge der Hautpflegezusammensetzung verglichen mit den umgebenden Bereichen des Topsheetmaterials enthält.

15. Absorbierender Artikel gemäß einem der vorhergehenden Ansprüche, **dadurch gekennzeichnet, daß** die Hautpflegezusammensetzung auf ein beliebiges Material und eine beliebige Komponente des Artikels appliziert wird, wie beispielsweise elastische Teile, Gürtel, Fasern usw., das/die während der Verwendung des Artikels in Kontakt mit der Haut der Trägers, beispielsweise über das flüssigkeitsdurchlässige Topsheet, steht.

16. Absorbierender Artikel gemäß einem der vorhergehenden Ansprüche, **dadurch gekennzeichnet, daß** mindestens zwei unterschiedliche Hautpflegezusammensetzungen in unterschiedlichen Bereichen des Artikels appliziert sind.

## Revendications

1. Article absorbant, tel qu'une couche, une couche-culotte, une protection anti-incontinence pour adulte, une serviette hygiénique ou un autre article semblable, comprenant une feuille de dessus (2), perméable aux liquides, une feuille de dos (3), imperméable aux liquides, et un corps absorbant (4) enfermé entre ces feuilles, lequel article contient une composition de soin pour la peau, qui est appliquée sur au moins une partie de l'article de sorte qu'elle puisse être transférée sur la peau du porteur de l'article, laquelle composition est solide à 21 °C et comprend au moins :
a) de 5 à 70 % en poids d'un composant dont le point de fusion se situe dans l'intervalle allant de 25 à 37 °C, choisi dans l'ensemble constitué par les cires synthétiques, paraffines, esters d'acides gras, esters d'acides gras polyhydroxylés, alcools gras, esters d'acides gras alcoxylés, et alcools gras alcoxylés, ainsi que les mélanges de tels composés ;
b) et de 5 à 70 % en poids d'un composant dont le point de fusion est plus élevé d'au moins 5 °C que celui du composant (a), lequel composant (b) est choisi dans l'ensemble constitué par les paraffines, esters d'acides gras polyhydroxylés, alcools gras en C₁₄₋₂₂, acides gras en C₁₂₋₂₂, et dérivés d'alcoxylation d'alcools gras ou d'esters gras, ainsi que les mélanges de tels composés ;
lequel article est **caractérisé en ce que** cette composition comprend en plus,
en tant que composant ;
c) de 5 à 25 % en poids d'un agent accélérateur de cristallisation, sous forme d'un poly(vinyl-stéaryl-éther), sous réserve que le point de fusion de cet agent accélérateur de cristallisation se situe dans l'intervalle allant de 45 à 70 °C,
et **en ce que** cette composition contient moins de 5 % en poids d'eau.

2. Article absorbant conforme à la revendication 1, **caractérisé en ce que** le point de fusion du composant (b) se situe dans l'intervalle allant de 40 à 60 °C.

3. Article absorbant conforme à la revendication 1 ou 2, **caractérisé en ce que** le composant (a) est choisi parmi les mélanges de glycérides d'acides gras comportant de 8 à 18 atomes de carbone, et de préférence, parmi des mélanges de qualité technique de glycérides partiels et/ou des mélanges contenant des glycérides.

4. Article absorbant conforme à l'une des revendications précédentes, **caractérisé en ce que** le composant (b) est choisi parmi les mélanges de glycérides d'acides gras comportant de 8 à 18 atomes de carbone, et de préférence, parmi des mélanges de qualité technique de glycérides partiels et/ou des mélanges contenant des glycérides.

5. Article absorbant conforme à l'une des revendications précédentes, **caractérisé en ce que** le composant (a) et/ou le composant (b) comprend ou comprennent chacun des triesters ou des esters partiels de glycérol et d'acides gras de coco, les mélanges présentant dans chaque cas un point de fusion situé dans l'intervalle indiqué.

6. Article absorbant conforme à l'une des revendications précédentes, **caractérisé en ce que** la composition de soin pour la peau comprend du composant (a) en une proportion pondérale de 10 à 60 %, du composant (b) en une proportion pondérale de 10 à 60 %, et du composant (c) en une proportion pondérale de 10 à 25 %.

7. Article absorbant conforme à l'une des revendications précédentes, **caractérisé en ce que** le point de fusion de la composition de soin pour la peau se trouve dans la gamme allant de 35 à 65 °C, de préférence dans la gamme allant de 35 à 50 °C, et en particulier dans la gamme allant de 35 à 45 °C.

8. Article absorbant conforme à l'une des revendications précédentes, **caractérisé en ce que** la composition de soin pour la peau comprend des cires de silicone en une proportion pondérale de 1 à 6 %, de préférence de 1,5 à 5,5 %, et en particulier de 2 à 5 %.

9. Article absorbant conforme à l'une des revendications précédentes, **caractérisé en ce que** la composition de soin pour la peau comprend des substances de soin pour la peau et/ou non-irritantes pour la peau en une proportion pondérale de 0,1 à 10 %, de préférence de 1 à 8 %, et en particulier de 2 à 6 %.

10. Article absorbant conforme à l'une des revendications précédentes, **caractérisé en ce que** la composition de soin pour la peau comprend de l'eau en une proportion pondérale de 0,5 à 3 %, de préférence de 0,5 à 2 %, et en particulier de 0,5 à 1,5 %.

11. Article absorbant conforme à l'une des revendications précédentes, **caractérisé en ce que** la composition de soin pour la peau comprend, en tant que composant (a), 50 à 60 % en poids d'un mélange d'esters de glycérol et d'acides gras de coco dont le point de fusion vaut de 30 à 33 °C, en tant que composant (b), 10 à 20 % en poids d'un alcool gras linéaire insaturé dont le point de fusion vaut de 57 à 60 °C, et en tant que composant (c), 15 à 20 parties d'un poly(vinyl-stéaryl-éther) dont le point de fusion vaut de 45 à 48 °C, ainsi que, en option, 2 à 5 % en poids d'une cire de silicone, et 5 à 10 % en poids d'une substance de soin pour la peau.

12. Article absorbant conforme à l'une des revendications précédentes, **caractérisé en ce que** la composition de soin pour la peau est appliquée sur au moins une partie de la feuille de dessus (2) de l'article.

13. Article absorbant conforme à la revendication 12, **caractérisé en ce que** la composition de soin pour la peau est appliquée en quantités différentes dans des zones différentes de la feuille de dessus.

14. Article absorbant conforme à la revendication 13, **caractérisé en ce que** la région de la feuille de dessus destinée à être mouillée ne contient pas de la composition de soin pour la peau, ou en contient moins que les régions environnantes de la feuille de dessus.

15. Article absorbant conforme à l'une des revendications précédentes, **caractérisé en ce que** la composition de soin pour la peau est appliquée sur tout matériau et tout composant de l'article, tels les éléments élastiques, ceintures, fibres, etc., qui sont en contact avec la peau du porteur de l'article au cours de l'utilisation de celui-ci, par exemple par l'intermédiaire de la feuille de dessus perméable aux liquides.

16. Article absorbant conforme à l'une des revendications précédentes, **caractérisé en ce qu'**il y a au moins deux compositions de soin pour la peau différentes qui sont appliquées dans des régions différentes de l'article.
